# EUROPEAN PATENT APPLICATION

(11) **EP 4 718 366 A1**
(43) Date of publication of application: **01.04.2026**
(21) Application number: 24202449.5
(22) Date of filing: 25.09.2024
(51) Int. Cl.: G06Q 50/12, A47J 44/00, G06F 40/103, G06N 3/02, G16H 20/60, G09B 19/00

(54) **GENERATING A CULINARY KNOWLEDGE BASE**

(71) Applicant: BSH Hausgeräte GmbH, 81739 München (DE)
(72) Inventor: Nakou-Franke, Gioninta, 83024 Rosenheim (DE); Cheong, Yi Heng, 440031 Singapore (SG); Kulkarni, Mohit, 414001 Ahmednagar (IN); Sagar, Kaushal, 573943 Singapore (SG)

(57) **Abstract**

A method (200) for generating a culinary knowledge base comprises steps of determining (210) user (140) actions during preparation of a dish; determining (205, 215) a first cooking recipe, the first cooking recipe comprising preparation steps which are similar to the user (140) actions; generating (220) a second cooking recipe, the second cooking recipe comprising preparation steps that match the user's (140) actions for preparing said dish; and storing (230) the second cooking recipe as a variant of the first cooking recipe.

## Description

The present invention concerns the generation of a culinary knowledge base. More specifically, the present invention may concern the automatic generation of user-created cooking recipes.

Cooking recipes for various dishes can be browsed and downloaded from dedicated recipe portals. A user may upload his or her own recipe creation to such a portal and possibly provide some meta-data like pictures, nutrition facts or allergens. However, such information is rarely consistent and correct. Fact-checking is rarely done and community-based feedback does not always work as expected. Many users may not try and improve, correct or vary a recipe in an online portal so that imperfect recipes may remain online for a long time. This may result in unattractive results when following a recipe from such a portal. Furthermore, it has been found that many recipes that may be of interest to other users remain undisclosed because users do not take the time and effort to formalize and upload them. A known recipe collection may be rich in irrelevant or incorrect information and comprise only limited culinary knowledge.

An object that underlies the present invention lies in providing a technical way for automatically generating an improved culinary knowledge base. The invention solves this object with the subject-matter of the enclosed independent claims. Dependent claims describe preferred embodiments.

It may be important to understand that methods, appliances and services mentioned herein may be used in complementing technologies, wherein one may be used to collect cooking data from users into a culinary database and the other to generate from collected culinary wisdom useful information to a user.

According to a first aspect of the present invention, a method for generating a culinary knowledge base comprises steps of determining user actions during preparation of a dish; determining a first cooking recipe, the first cooking recipe comprising preparation steps which are similar to the user actions; generating a second cooking recipe, the second cooking recipe comprising preparation steps that match the user's actions for preparing said dish; and storing the second cooking recipe as a variant of the first cooking recipe.

The present invention can and is preferred to be used with a number of different dishes. In fact, it is expected that the presently envisaged technology will function better and better the more recipes for one or more dishes are collected. Said technology is also preferred to be employed with many users so that cooking habits or preferences of many different persons may be retrieved, condensed and stored.

The culinary knowledge base may also be called culinary database, cooking database, recipe repository or similar. The knowledge base may be filled with information about a large number of cooking recipes and information contained in these recipes may be made available to one or more users. As will be explained in more detail below, the culinary knowledge base may later be used to automatically provide suggestions, hints or proposals to a user who wishes to prepare a dish.

The knowledge base may thus be populated with cooking recipes which are clustered by similarity so that correlating recipes share a relationship. Variants for the same dish may thus be more easily detectable. By applying a similarity criterion, it can be determined just how much difference lies between two cooking recipes. Even a tiny variation like a variation in the amount of an ingredient may be tracked. Such a variation may have a big impact (e.g. saffron in a saffron cake) or a small impact (e.g. sugar in a tomato sauce). Based on the measure (or unit) for similarity, a large number of recipes can be interrelated, even though the culinary impact of their differences may not be obvious. A broad range of variations for one dish can thus be collected. Regional, personal or inter-family variations may be tracked and stored for later use. This may help to prevent that recipes for a dish deteriorate into a few main-stream variants. Instead, a multitude of co-existing recipes for one dish may be kept and made use of.

According to one embodiment of present method, it is determined that a dissimilarity between the first and second cooking recipe is lower than a first predetermined threshold. This allows to accept second recipes only if they are similar enough to the first recipe to count as a variation. A second recipe that differs from the first one significantly and/or in several parameters may thus be considered a separate recipe on its own and may be stored accordingly. It may also be determined that the second recipe resembles another known recipe more closely than the first recipe. The second recipe may then be stored as a variant of said known recipe. The second recipe may also be stored as a variant of more than one known recipe. Variants may work both ways so that if a second recipe is a variant of a first recipe, the first recipe may also be considered a variant of the second one.

Similarly, it may be determined that a dissimilarity between the first and second cooking recipe is greater than a second predetermined threshold. This allows to accept only second recipes as variants, which differ considerably from the first recipe. Recipes that are almost identical may be considered basically the same thing.

The first and second thresholds may be used in combination so that second recipes may be accepted and stored if they are different enough yet not too different from the first recipe. The first threshold needs to be larger than the second threshold for this to work.

Each recipe may be associated with a point in a latent space of an artificial neural network (ANN); and a dissimilarity between two recipes may be determined on the basis of a distance between their associated points in said latent space. The ANN may especially comprise a feedforward neural network (FNN) and the network may comprise neurons in several layers, wherein some of the layers may be hidden. The ANN may be trained to associate to each recipe a point in said latent space. A point may be described by a vector with components that reflect e.g. preparation steps or ingredients of a recipe. The dimensionality of a cooking recipe may therefore be large and cooking recipes may have different dimensionalities. The network may still be able to determine a dissimilarity between them. Missing information in some categories (that is: elements of said vector) may not be problematic. Transformer technology, such as a large language model, may be used. The transformer may use multi-head-attention. Position embeddings and/or positional encoding may be used.

It is a general advantage of ANNs to be able to determine an output vector with a low dimensionality on the basis of an input vector with a high dimensionality. This property may be exploited for fast and reliable determination of dissimilarity between cooking recipes. This reduction effect can be easily experienced with an example ANN that is trained to recognize the picture of a cat. The input vector may comprise all picture elements (pixels) of an input picture and thus be very large, while the output vector may be as small as one element which outputs whether or not the picture seems to contain a cat.

The ANN may be intentionally trained to assign points to recipes in such a way that said distance between assigned points reflects a culinary difference between dishes. During training, weights and biases for neurons of the ANN may be gradually adjusted such that this condition is achieved more and more closely. A ground value of "culinary distance" for the training data may be provided by a culinary expert, possibly on the basis of tasting or otherwise culinary judging the end product of a recipe, that is, a prepared dish.

It is another property of a trained ANN to be able to successfully process input data that does not match any of the data that was used for its training. This allows the ANN to determine a point in the latent space for a recipe that is novel over all training data. Training data is preferred to cover a large spectrum of culinary diversity and the density of recipes in the latent space should not be too low to provide satisfactory results when assigning latent space points to recipes.

A dissimilarity between points is preferred to be normalized to some predetermined scale like from 0 to 1 or from 1 to 10. An opposite of a dissimilarity may be considered a similarity (and vice versa). Similar recipes may exhibit a small distance between associated points in latent space and dissimilar ones a larger distance. A user may choose to work on similarities or on dissimilarities.

Various measures for distances between points in the multi-dimensional latent space may be applied. According to one embodiment, an Euclidean distance may be calculated. According to another embodiment, cosine similarity may be used. Other exemplary distances between data points comprise Manhattan, Minkowski or Chebyshev distances. In machine learning, common kernel functions such as the real basis function kernel (RBF kernel) can be viewed as similarity functions.

The first cooking recipe may be selected from a repository or database comprising a plurality of cooking recipes. The second recipe may later be stored in that same repository. The first recipe may be selected before the preparation of the dish starts and it is usually the user carrying out the preparation who chooses the first cooking recipe. In the moment, in which the user's actions deviate from the steps of the first recipe, a second recipe is automatically generated.

It is preferred that the second recipe will be generated after the user has finished preparation; or a second recipe determined throughout the process of cooking may be updated when or until the user is done with cooking. In some embodiments, the user may start from a predetermined first recipe and vary it during cooking.

In some embodiments, the user does not actively select a first recipe in the first place. Instead, he or she performs cooking actions which are detected and evaluated. A first recipe that conforms to the cooking actions may be found in a repository with many cooking recipes. This allows the user to perform free or improvised cooking. From the repository, more than one first recipes may be retrieved that match the performed cooking steps to some degree. A first recipe may be selected that exhibits the highest resemblance to the performed cooking steps. That is, the first cooking recipe may be determined as the one that is most similar to the second one.

The first recipe may be generated in text form using a Large Language Model (LLM). The LLM may be multi modal and accept input data in various forms such as integer or floating point measurements, pictures or sounds. The ANN may be adapted to determine similarities between recipes in text form.

The user action may concern an ingredient or an intermediate product of the dish, or an amount or state of the ingredient or intermediate product. Property data may be determined using a scale or a volumetric measurement unit. The ingredient or intermediate product may be determined by a user input. There may also be provided a camera for taking pictures during the preparation process; and an ingredient or an intermediate product or its state may be determined on the basis of one or more pictures. For this, there may be provided a mechanism for object recognition based on pictures. In case an LLM is used for generating the second recipe, the LLM may be adapted to directly work with pictures as input.

The user action may also comprise processing of an ingredient or intermediate product. Such processing may comprise e.g. cutting, heating, cooling, stirring, opening, squeezing, shredding or kneading. Processing information may be determined on the basis of an observation of the user's actions, especially if a camera is provided.

The user action may comprise using one or more kitchen appliances. Several kitchen appliances may be operated together, e.g. a stove and a mixer; or a blender and a refrigerator. Processing may be determined on the basis of operation information of the one or more appliances. Appliance operation may be further characterized by information like time, appliance settings, user operation input or process data. For instance, a kitchen appliance may knead dough and an electric current flowing through an electric motor may be measured and the measurement may be provided as operational information. Such measurements may allow inference of a consistency or mass that is treated. Other measurements may comprise a processing duration, a processing intensity or a target setting for the appliance.

The appliance may comprise sensors for determining a status of an ingredient or intermediate product. There may, for instance, be provided a scale with which an amount of matter added into a processing container may be determined.

The user may employ an integrated kitchen appliance that is adapted to perform various types of processing of food ingredients or intermediate products. One exemplary kitchen appliance of this kind is known as Cooklt. Some of the types of food processing, which it can perform, require an additional tool or extension. The appliance may be adapted to detect an installed tool or extension and provide this data for characterizing a processing step during preparation of said dish. The integrated kitchen appliance may be operated standalone or in combination with one or more other kitchen appliances.

It is preferred that performed processing steps are determined automatically. This may concern processing steps the user performs, such as adding, cutting or stirring, as well as processing steps that are performed by a kitchen appliance, such as heating, stirring, cooling, kneading, mixing, shredding etc. In some cases, combined steps may be detected such as the user inserting dough on a cake tin into an oven that is pre-heated to a certain temperature. Optionally, a setting or configuration of the oven may be detected, such as a height on which the dish is installed inside the cooking chamber, a heating mode (e.g. top heat, bottom heat, air circulation) or an operation mode (e.g. steaming, heating, grilling).

The second recipe may be stored as a variant of the first recipe if the user expresses his contentment with the prepared dish. The user may express his or her contentment by inputting it via a user interface. It can be inferred that the user is satisfied with the outcome of the recipe if he/she prepares it repeatedly. The second recipe may be stored in such a way that it cannot be actively selected as a first recipe for some time. The newly stored recipe may be assigned a predetermined level of contentment or popularity. Should the second recipe (or recipes that differ no more than a predetermined amount from it) be prepared repeatedly, the level of contentment or popularity may be increased. As the level exceeds a predetermined threshold, the second recipe may be related as variant to one or more known recipes in the database. In one embodiment, similarity relationships between recipes in the database are determined or adjusted on a regular basis. A second recipe with a high enough popularity may be selectable as a first recipe.

The second recipe may be stored with a reference to characteristics of a user who prepared it. Users with similar characteristics may be assigned to a group and the second recipe may be assigned to one or more of such groups. Recipes may thus be clustered by user characteristics. Assigning a user to a recipe may be done on the basis of statistical tools like a naive Bayes filter. Alternatively, machine learning may be employed to determine a relationship between a recipe and characteristics of users who prepare dishes according to said recipe. A set of characteristics may collectively be called a persona. Several persons may have the same persona.

The user characteristics may have one or several dimensions or categories. In some embodiments, the characteristics comprise culinary preferences of said user. This may include dietary preferences like halal or kosher food, salt-free, glutenfree, sugar-free or fat-reduced food. It may also comprise a predetermined food regimen or the avoidance of certain allergens. The culinary preferences may also reflect habits or preferences for preparing food. A user may prefer fried food over baked food, simple preparation over intricate preparation or he may focus on the use of inexpensive ingredients or on short preparation times. The user may be more or less experienced in the preparation of food and choose recipes according to his or her level of proficiency. Such preferences may vary and/or develop over time.

The characteristics of said user may comprise a history of dishes, which the user has prepared before. The history may be automatically determined based on the observation of the user during food preparation. Historic information may be collected over extended time periods and trends may be detected.

The characteristics of said user may comprise personal information of the user. The user may choose to enter such information at a web service so that adapted suggestions may be produced for him or her. Some information may also be determined automatically, such as a location or time zone the user resides in. Other personal information may comprise gender, age, ethnicity, religion, family status, political attitude or income.

Similarity or dissimilarity between two recipes may be determined with associated user characteristics in mind. For instance, a first recipe, which is favoured by a first group of users with some predetermined first user characteristics, may be determined to be dissimilar to a second recipe, which is favoured by a second group of users with some predetermined second user characteristics, if the user group characteristics differ from each other.

In some embodiments, a recipe may be assigned a point in the latent space of an ANN on the basis of a combination of recipe properties and user characteristics of an assigned user. Recipe similarity may thus encompass similarity between user or group characteristics of associated users. Also, similarity or dissimilarity between recipes may vary for different users.

In some embodiments, a second recipe may be stored as variant of a first recipe only if the user who prepared the second recipe more or less matches a user group that is associated to the first recipe. This may later allow determining better recommendations to a user in search of a recipe.

According to another aspect of present invention, a kitchen appliance comprises a sensor for determining a user action during preparation of a dish; and processing means. The processing means are adapted to determine, based on determined user actions, a first cooking recipe, the first cooking recipe comprising preparation steps which are similar to the user actions; to generate a second cooking recipe, the second cooking recipe comprising preparation steps that match the user's actions for preparing said dish; and store the second cooking recipe as a variant of the first cooking recipe.

The processing means may be adapted to carry out, completely or in part, a method disclosed herein. The processing means may be of electronic nature and may comprise a micro-computer or micro-controller, an ASIC or a similar device. The method may be realised as a computer program product with program code means and may be stored on a computer readable medium. Features or advantages of the method may be applicable to a corresponding device or system as well as vice versa.

According to yet another embodiment of present invention, a service is adapted to determine dissimilarity between two cooking recipes on the basis of an artificial neural network. The kitchen appliance may comprise a communication unit for exchanging data with the remote service. The remote service may comprise a collection of first cooking recipes and a user of the kitchen appliance may be allowed to select a first recipe from said collection. The service may be adapted to accept and answer requests from different kitchen appliances in different households. It is preferred that the service is implemented in a computer cloud or in a server. The service may also be adapted to manage other data of a user, an associated household appliance or an associated household. Data of users living in the same household may be linked to each other.

A system for generating a culinary knowledge base may comprise said kitchen appliance and a remote service. The system may comprise more than one kitchen appliance in the same household. The system may be adapted to carry out a method disclosed herein completely or in part.

The culinary knowledge base may be used for automatically generating suggestions, hints or support information to a user for preparing a dish. Details or features may be shared between the generation and the use of the culinary database described herein.

For instance, a method for providing guidance to a user in preparation of a dish comprises steps of determining a first cooking recipe, the first cooking recipe comprising preparation steps for preparing said dish; determining user actions in preparation of said dish; generating a second cooking recipe, the second cooking recipe comprising preparation steps that match the user actions more closely than the first cooking recipe; and offering the user to switch from the first to the second cooking recipe.

It may be determined that a dissimilarity between the first and second cooking recipe is lower than a predetermined threshold. It may also be determined that the dissimilarity is higher than another predetermined threshold.

The first and second cooking recipes may be selected from a repository comprising a plurality of cooking recipes. The first cooking recipe may be selected by a user who then carries out food preparation. The second cooking recipe may be automatically determined from the same repository. The repository may especially be built on the basis of a technique described above.

Cooking recipes that correspond to the same dish may be grouped; and both recipes may share the same group. For example, a cooking recipe for pancakes may be grouped with other recipes for pancakes and not with a recipe for waffles, even though there may be a certain similarity.

The user action may, for example, concern an ingredient or intermediate product of the dish, or an amount or state of the ingredient or intermediate product. The user action may also comprise a processing of an ingredient or intermediate product. The user action may also comprise the use of one or more kitchen appliances. A kind or mode of operation of the kitchen appliance, an installed extension or tool, a time or duration, an intensity or other operational data of the kitchen appliance may be comprised by the user action. The user action is preferred to be determined automatically. For this, one or more sensors may be employed and/or operation data of one or more kitchen appliances that are used in the process of preparing said dish may be collected.

In some embodiments, the second recipe is determined on the basis of its popularity. Popularity may be determined based on the number of times the recipe was downloaded or used as a basis for preparation of the associated dish. Popularity may also be determined on the basis of expressed user feedback. Optionally, only feedback of a user who is known to have at least attempted to prepare a dish according to a recipe may be recorded. More specifically, user feedback may be restricted to recipes the user has prepared.

The second recipe may be determined on the basis of a similarity of a characteristics of the user and characteristics of other users who have prepared a dish according to the second recipe. Recipes in a database or repository may be associated with one or more users or with one or more groups of users. A group of users groups together users with similar characteristics. A recipe in the database may only be proposed as a second recipe to a user if the user's characteristics are similar enough to characteristics of at least one of the users or groups associated to the recipe. Different users may thus be offered different recipes as second recipe.

The characteristics may comprise culinary preferences of that user. User characteristics may also comprise a history of dishes the user has prepared before. Furthermore, characteristics of a user may comprise personal information of that user.

A kitchen appliance comprises a sensor for determining a user action during preparation of a dish; a user interface; and processing means. The processing means are adapted to determine a first cooking recipe, the first cooking recipe comprising preparation steps for preparing said dish; to determine user actions in preparation of said dish; to generate a second cooking recipe, the second cooking recipe comprising preparation steps that match the user actions more closely than the first cooking recipe; and to offer the user to switch from the first to the second cooking recipe.

A service may be adapted to determine dissimilarity between two cooking recipes on the basis of an artificial neural network. A system for providing guidance to a user in preparation of a dish may comprise the above-mentioned kitchen appliance and the above-mentioned service.

Non-limiting embodiments of the invention will now be discussed in more detail with reference to the enclosed drawings in which:
- figure 1: shows a system;
- figure 2: shows a flow diagram of a first method;
- figure 3: shows a flow diagram of a second method;
- figure 4: shows a proposed method for processing similarities;
- figure 5: shows an example for embedding processing; and
- figure 6: shows a schematic representation of embeddings in determining similarity.

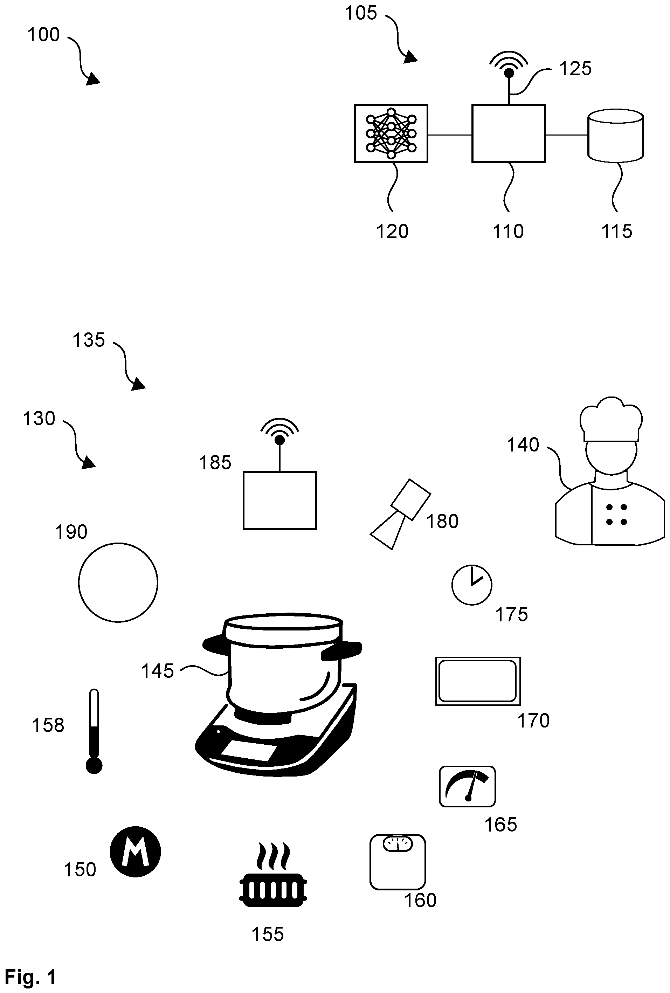

Figure 1 shows an embodiment of a system 100 for creating and using a culinary knowledge base 105. The culinary knowledge base 105 may function as a database, in which information can be stored and stored information can be retrieved on the basis of predetermined search criteria. In the present embodiment, the knowledge base 105 is implemented on hardware that comprises processing means 110, a storage 115, an artificial neural network ANN 120 and communication means 125. Said hardware may be installed in a central place like a datacenter or a server installation. This hardware may be adapted to communicate with several households 130 in a way that will be explained hereinafter.

The system 100 furthermore comprises a kitchen appliance 135 in a household 130. Although Fig. 1 shows only one kitchen appliance 135, the present invention encompasses the use of several kitchen appliances 135 in the same household 130. Exemplary household appliances 135 comprise a mixer, a stove, an oven, a refrigerator, a cutter, a kneader, a grinder or a shredder. The shown kitchen appliance 135 is a combined appliance which integrates functions of several other appliances 135 into one.

As one part of the present invention, it is proposed to monitor the actions of a user 145 while he or she prepares a dish. Monitoring may comprise collecting operating data of one or more kitchen appliances 135 such as a setting, a configuration or a user input. In addition, there may be provided one or more sensors that are adapted to collect data concerning the cooking process. A sensor may be integrated into a kitchen appliance 135 or come as an external sensor. Several sensors may be integrated into one device.

The exemplary kitchen appliance 135 of Figure 1 has a receptacle 145 in which processing takes place. There may be provided a motor 150 for powering gear like stirring, cutting or kneading devices which may be configurable with the kitchen appliance 135. From the motor 150 data like a rotational speed, a current consumption of a configuration with gear may be collected. There may also be a heater 155 that is configured to heat contents of said receptacle 145. The heater 155 may yield data like a current temperature, a set temperature or an electric current flowing through the heater 155. In another variant there may be provided a cooler for contents of the receptacle 145 and it may yield similar data. A temperature of the receptacle 145 or its contents may also be determined with a temperature sensor 158.

There may be provided a scale 160 that is adapted to weigh contents of the receptacle 145 or an ingredient or intermediate product that is added to (or taken out of) the receptacle 145. It is preferred that the scale 160 is integrated into the appliance 135, but a standalone version may also be used. Similarly, there may be means for measuring volumes, especially of liquid or fluid material in, into or out of the receptacle 145. Such means may comprise a measuring cup or similar.

A user interface 165 may be provided for permitting the user 140 to set, control or verify operational parameters of the kitchen appliance 135. The user interface 165 may comprise one or more input elements like knobs or switches and/or one or more output elements like control lamps, analog or digital dials or the like. Input and output elements may be combined, for example in a touch screen.

An electronic cookbook 170 may be integrated into a kitchen appliance or come as a separate device. In one embodiment the cookbook 170 is implemented as an app on a smartphone, a tablet computer or a similar device. The cookbook 170 may permit the user 140 to select a cooking recipe from a repository and have processing steps and other information displayed. Usually, the cookbook 170 leads the user 140 step by step through the processing required for preparing a dish. The cookbook 170 may permit interaction such as searching or browsing through the recipe repository or scaling a recipe to a desired number of servings.

A timer 175 may be provided to give an alarm after a predetermined time has passed. This may help the user 140 to process an ingredient or intermediate product for the right time. The timer 175 may also be used to supply timing information so that for instance it can be recorded when or for how long the motor 150 or the heater 155 was powered on.

An optional camera 180 is adapted to monitor actions of the user 140 and/or events comprising the kitchen appliance 135, especially the receptacle 145 or its contents. The camera 180 may be adapted to recognize an object and further optionally to provide object data like a size, weight or state of a recognized object.

Communication means 185 are adapted to communicate with the culinary knowledge base 105. In Figure 1, these means 185 are depicted as a wireless interface, however other kinds of connection may also be used. In some embodiments the culinary knowledge base 105 is located in the household 130. The culinary knowledge base 105 may be part of a household appliance 135, in which case communication means 125, 185 may not be necessary.

Processing means 190 are adapted to process cooking information on the side of the household 130. The processing means 190 are preferred to be connected to one or several of the aforementioned sensors and/or actors. Especially in case of more than one household appliance 135 there may be a plurality of processing means 190 which are then preferred to be communicatively connected such as to share data and information. Should the culinary knowledge base 105 be local to the kitchen appliance 135, processing means 110, 190 may be comprised by one integrated unit.

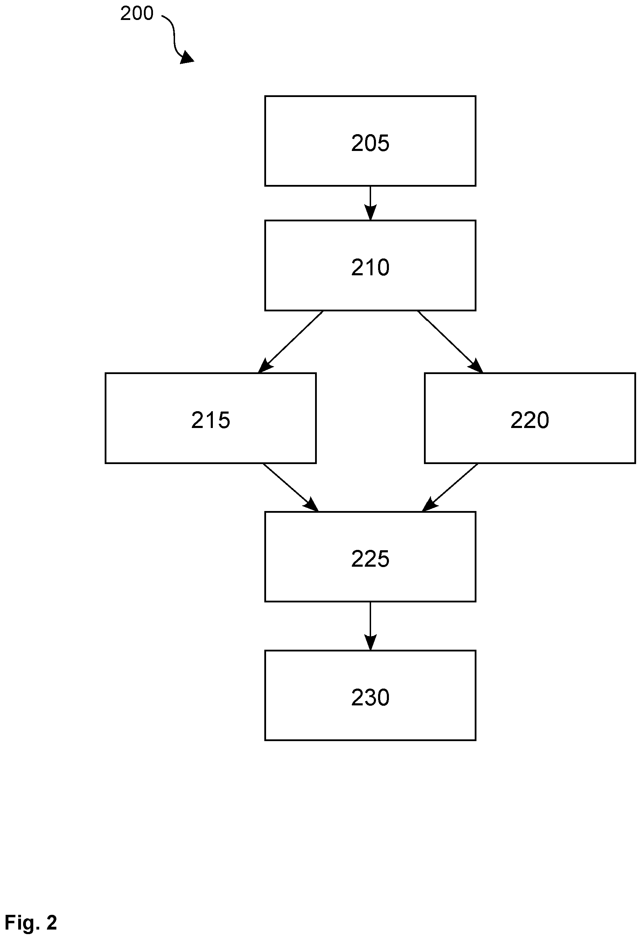

Figure 2 shows a flow diagram of an embodiment of a method 200 for generating, extending and/or improving a culinary knowledge base 105. In a step 205, a first cooking recipe may be selected. The first recipe may be selected from the culinary knowledge base 105 and selection may be done be a user 140 who may later carry out processing steps for cooking a dish by following instructions contained by the first recipe.

A cooking recipe may comprise information on ingredients to use and steps to take for preparation of a predetermined dish. The preparation steps may include information about actions to take or processing to carry out. Some processing, especially heating, may be done with the help of a kitchen appliance 135. Actions may especially comprise preparing or combining ingredients or intermediate products.

In a step 210, cooking steps actually carried out in preparation of said dish may be determined. This is preferred to be done automatically by observing settings or processing data from one or more kitchen appliances 135. Additionally, data from a sensor that scans a workspace for preparing the dish can be evaluated. Optionally, the user 140 may enter information on what he or she is doing, either in text format or with spoken words, which may be automatically recognized and turned into processable or storable data, especially text data. Spoken words may be turned into text with a suitable speech-to-text technology such as a large language model LLM with multi-modal capabilities. As will be shown later, all data may be brought into a predetermined format that is suitable for comparing or relating recipes.

Should no first recipe have been set prior to cooking, a first recipe may be selected from a cooking recipe repository such as the culinary knowledge base 105 in a step 215. Steps that have been carried out so far may be matched against steps of recipes in the repository. The first recipe may especially be determined on the basis of a similarity between the determined steps and steps of a stored recipe.

The first recipe may comprise cooking steps that do not perfectly match the determined cooking steps. Instead, a likely first recipe may be determined. Likeliness may be determined on the basis of various parameters. As will be shown in more detail below, such parameters may comprise popularity of a dish. Also, characteristics of the user 130 may be compared to characteristics of one or several users 130 which are known to appreciate a recipe.

In a step 220, a second cooking recipe may be determined, wherein the second recipe strictly follows the previously observed steps. The second recipe may be determined after cooking is done as it is preferred to comprise all steps that were actually carried out.

It is preferred that in a step 225 it is determined that the user 130 is satisfied with the outcome of the cooking that he or she performed. The user 130 may manually give an indication of how happy he or she is with the cooking result or another user 130 may assess the prepared dish and provide an evaluation. Unless the evaluation hints at a failed cooking experiment, the method 200 may continue.

In a step 230 the second recipe may be stored as a variant of the first recipe. The first and second recipes may contain instructions for cooking the same dish in different variations. Variants of the same dish may be required to differ by a predetermined amount from an existing variant. There may be minimum and/or maximum differences between the second and the first recipe for the second recipe to be accepted as a variant. Should the second recipe match closely enough a second recipe that is already known as a variant of the first recipe, the second recipes may be considered the same.

After method 200 has been carried out, the culinary knowledge base 105 may be extended by one more recipe which has a relationship to at least one other recipe in the knowledge base 105. Next time a user 130 browses the culinary knowledge base 105 in search of a recipe for a dish he or she may come across the newly stored variant and choose to follow its instructions for preparation of said dish.

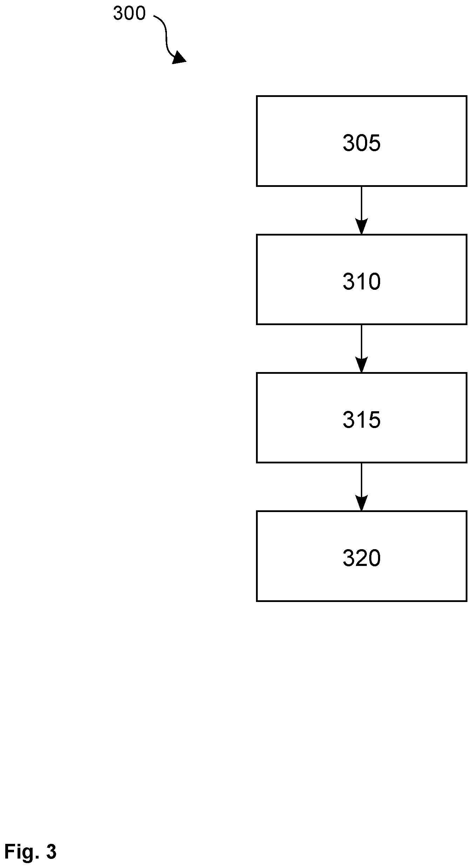

Figure 3 shows a flow diagram of an embodiment of a method 300 for providing culinary guidance to a user 130 in preparation of a dish. In a step 305, a first cooking recipe may be selected. The first recipe may be selected from the culinary knowledge base 105 and the selection may be done by a user 140 who may later carry out processing steps for cooking a dish by following instructions contained by the first recipe.

In a step 310, cooking steps actually carried out in preparation of said dish may be determined. This is preferred to be done automatically by observing settings or processing data from one or more kitchen appliances 135. Additionally, data from a sensor that scans a workspace for preparing the dish can be evaluated. Optionally, the user 140 may enter information on what he or she is doing, either in text format or with spoken words which may be automatically recognized and turned into processable or storable data, especially text data. Spoken words may be turned into text with a suitable speech-to-text technology such as a large language model LLM with multi-modal capabilities. As will be shown later, all data may be brought into a predetermined format that is suitable for comparing or relating recipes. Note that step 305 may correspond to step 205 and step 310 may correspond to step 210 of method 200.

In a step 315, a second cooking recipe may be determined, wherein the second recipe matches the previously observed steps more closely than the first cooking recipe. In other words, should the cooking steps, which the user 130 performs while preparing said dish, deviate from the steps given in the first cooking recipe, a second cooking recipe may be retrieved from the culinary knowledge base 105, wherein the second cooking recipe matches the steps carried out so far more closely than the first cooking recipe. Should no such second recipe be found in the culinary knowledge base 105, a warning can be provided that the current cooking process appears to be deviating from the instructions comprised by the first cooking recipe.

The user 130 may be offered information from the second recipe in order to continue his or her course of preparing the dish. In one embodiment, the user 130 may be suggested to switch from the first to the second cooking recipe. Should the user 130 accept this suggestion, he or she may build on the work already carried out so far and still produce the dish, albeit in a different way. In another embodiment, information from the second cooking recipe may be provided to the user 130 alongside information from the first cooking recipe. In this way, the user 130 may continue to deviate or improvise on the basis of the first cooking recipe but with the additional knowledge of the second recipe. Naturally, more than one second recipe may be found and information from more than one recipe may be provided to the user 130.

The present invention builds upon the ability to determine a similarity between two cooking recipes. This may be used to determine if one recipe matches another recipe, how similar one cooking recipe is to another one or if a cooking recipe is more similar to one recipe than to another recipe. Determination of similarity may also be used for a similarity search. For this, incomplete recipe information may be treated as an incomplete recipe and there may be determined a known cooking recipe with a maximum similarity.

It is proposed to determine similarity between two cooking recipes by comparing associated vectors. In this, a vector comprises several components, each of which may be determined on the basis of e.g. a preparation step or an ingredient of a recipe.

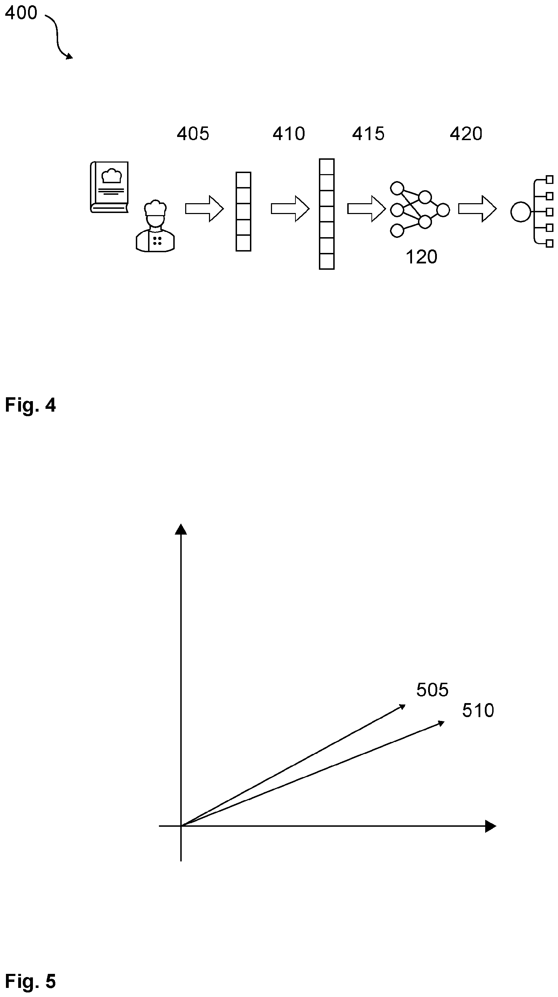

Figure 4 shows a proposed method for processing similarities. Method 400 starts with actions or observations of steps carried out by this user 130. Collection of this kind of data may correspond to a step 210 or 310 in methods 200 or 300, respectively.

In a step 405, this data may be converted into tokens. Each recipe is preferred to be accompanied by additional information, such as difficulty level, main ingredients, regional cuisine, season, preparation time, and more. These pieces of information are referred to as keywords, and they are defined for each recipe. Additionally, to enhance our system's capabilities, more keywords may be defined, for instance based on user behaviour, such as step modifications, ingredient changes, step repetitions or skips, and actual cooking time. These user-generated keywords can be added to the corresponding recipe. In natural language processing (NLP), we refer to these keywords as tokens. Tokens are individual units of text that are used in NLP and machine learning tasks. In the context of NLP, tokens can refer to words, characters, or even subwords, depending on the specific task and tokenization technique used. In our case we are using a Word Tokenization technique.

Exemplary tokens comprise a time, at which the step was carried out, a duration or an amount of an ingredient that was added into the receptacle 145. There may be a number of predefined tokens and each action may be categorized according to the available tokens. It is preferred that the tokens comprise natural language or human-readable text.

Implementing a machine learning algorithm with words is not possible. For this reason it is proposed to convert those tokens to vectors. These vectors (embeddings or representations) capture semantic and contextual information about the words or phrases, enabling machine learning models to understand and process natural language more effectively. Similar recipes lead to similar vectors.

Vectorizing the tokens can be done with many techniques. The simple models like word2Vec or GloVe capture some semantic relationships between words where the more complex methods such as Universal Sentence Encoder, BERT and large language methods like Llama, Falcon7B and StableBeluga2 extend this concept to represent longer sequences of words or entire sentences. These embeddings capture not only the meaning of individual words but also the relationships and context within the larger linguistic unit. The disclosure here is preferred to use natural language embeddings for recipe steps which are a naturally structured, sequential form of instructions.

From the user interaction feature tokens, a representation embedding can be generated for each token in a step 410. The embedding is a high dimensional feature vector that represents the interaction feature token and hence the user behaviour in the embedding space. In this way, user behaviours can be observed in a mathematical framework and different behaviours can be compared.

The similarity of vectors can be measured using various methods, such as the cosine function (cosine similarity) or the Euclidean distance. The cosine function is used to measure the similarity between two vectors in a multi-dimensional space. Based on this, we can determine if the vectors are similar. In our case, we can apply these methods to measure the similarity of recipes, not only in a general sense, but also at a more specific level, such as comparing individual steps. Also here we can use some methods to reduce the dimensionality of the Vectors with methods like PCA (principal component analysis), t-SNE (t- Distributed Stochastic Neighbour Embedding), LLE (Locally Linear Embedding) or Auto-Encoders. The aims of those methods is to transform a high-dimensional dataset into a lower-dimensional space while preserving the most important information and minimizing the loss function.

In a step 415, said embeddings can be forwarded into an ML pipeline which may process the embeddings and yield a result in a step 420.

Figure 5 shows an example for embedding processing according to an embodiment of the present invention. A first exemplary cooking recipe 505 for Mousse au Chocolat comprises exemplary keywords "advanced", "cookitrecipe", "dessert", "french", "glutenfree", "lowcarb", "party", "preparationTimeMax15" and "totalTime-Max60". A second exemplary cooking recipe 510 for Creme Caramel comprises exemplary keywords "bakesweet", "cookitrecipe", "dessert", "easy", "french", "preparationTimeMax15" and "vegetarian".

Although a human may be able to estimate similarity between the two cooking recipes 505, 510, determining a mathematical value for similarity is difficult as this data representation does not capture relationships between different items with high resolution. It is therefore proposed to transpose the keyword into components of a vector for each of the recipes.

In the given example, the first cooking recipe 505 may be assigned a vector with components [-0.03223406, 0.12216095, 0.25707862, -0.09802143, 0.34227863,...] while the second cooking recipe 510 may be assigned a vector with components [-0.0503796004, 0.293493629, -0.125535294, 0.19743263, - 0.288338900...].

Each vector defines a point in latent space and a distance between the points corresponds to a dissimilarity between the respective cooking recipes. The simplified graphic of figure 5 shows that corresponding end points of the corresponding vectors enclose a certain distance between them. If the distance is small, dissimilarity is low and similarity is high. If the distance is large, dissimilarity is high and similarity is low. Distances may be determined using different approaches. The determined distance may be normalized to a predetermined scale to allow an estimation of how similar two cooking recipes are to each other. The distance may also be compared to a predetermined threshold. This approach permits to compare all possible cooking recipes using the same framework.

It is furthermore proposed to extend the generation of vectors to include characteristics of a user 130 or group of users 130 that is associated with a cooking recipe. Characteristics of a user 130 or group may correspond to predetermined categories. From the embeddings of the user generated tokens and the embeddings of the recipe steps, these are inputs into a machine learning pipeline with a predictive task to classify users according to some prerequisite. Example categories of users 130 may be (but not be limited to) more or less experienced, exploratory, minimizing, time conserving, users 130 who favour some types of cooking steps over others, or users with a particular nutrition goal in mind.

Additionally, the use of embeddings can be expanded beyond just representing user interaction features or recipe steps. Recipe specific data may also be embedded. Some examples of additional embeddings that can be generated include the nutrition quality of a recipe, the types of changes of nutrition made by user actions, or intention feature tokens of users among others.

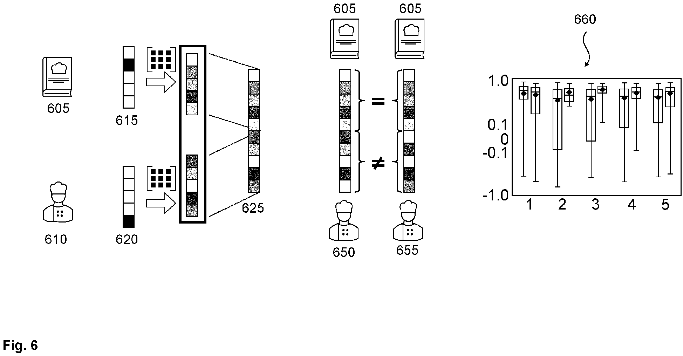

Figure 6 shows a schematic representation of embeddings in determining similarity according to the present invention.

Recipe steps 605 and human actions are independently assessed. Recipe steps may comprise information such as "separate three eggs and put the whites in the receptacle" or "add 30 g of sugar". Human actions may comprise the addition of an extra ingredient, the skipping of one or more steps or extending the cooking time given in the recipe steps 605.

Next, recipe tokens 615 are generated based on the recipe steps 605; and human interaction feature tokens 620 are generated based on the human actions 610.

On the basis of the trained ANN 120, a user behaviour defined embedding may be produced. The recipe tokens 615 may be turned into a first vector of recipe embeddings and the human interaction feature tokens 620 may be turned into a second vector of human interaction feature embeddings. A concatenation of the generated vectors may represent the user behaviour-defined embedding 625.

Characteristics of a user 130 may also be used in the embedding 625. Should a first user 650 and a second user 655 both work on the same recipe step 605, the corresponding vectors may differ in the part that represents the user characteristics. On a group level, similarities between distributions between user groups working on the same recipe step may be compared. In Figure 6, this is visualized with a candlestick chart 660. A horizontal direction shows recipe steps and a vertical direction shows similarities. Left candlesticks for each step correspond to a first user group and right candlesticks to a second user group. The first user 640 may be in the first group and the second user 655 may be in the second group. The user groups are preferred to be chosen such that the variation between user-related embeddings is low.

The ANN 120 is trained such that recipes, recipe steps, human feature interactions or user characteristics that are similar will lead to points in lateral space that are close together. Through the training process of the ANN 120, the embedding weights of recipe tokens, the human interaction feature tokens, or both, are updated to improve the classification accuracy of the model. Training may use labelled data. If the model that is represented through the ANN 120 reaches a certain degree of accuracy, individual and group differences can appear when the embeddings of each group of users are generated and clustered.

Within the population of each group, the sum of differences of the user behaviour defined embeddings shows the steps in which a statistically significant population difference occurs. This is calculated using a distance measure between high dimension vectors, which may be a cosine similarity, an Euclidean distance or another distance measure.

After training, clustering of the human interaction feature tokens 620 alongside the recipe step tokens 615 in the principal component analysis space would result in human interaction feature tokens 620 more closely mapped to the recipe step tokens 615 that they occur together with.

### Reference list (as part of the description)

- 100: system
- 105: culinary knowledge base
- 110: processing means
- 115: storage
- 120: artificial neural network
- 125: communication means

- 130: household
- 135: kitchen appliance
- 140: user
- 145: receptacle

- 150: motor
- 155: heater
- 158: temperature sensor
- 160: scale
- 165: user interface
- 170: electronic cookbook
- 175: timer
- 180: camera
- 185: communication means
- 190: processing means

- 200: method
- 205: select first cooking recipe
- 210: determine cooking steps carried out
- 215: determine first cooking recipe
- 220: determine second cooking recipe
- 225: determine user contentment with outcome
- 230: store second cooking recipe as variant of first cooking recipe

- 300: method
- 305: select first cooking recipe
- 310: determine cooking steps carried out
- 315: determine second cooking recipe
- 320: offer information from second cooking recipe for continuing cooking

- 400: method
- 405: convert into tokens
- 410: generate representation embedding
- 415: forward embeddings to ML
- 420: provide result

- 505: first cooking recipe
- 510: second cooking recipe

- 600: process
- 605: recipe steps
- 610: human actions
- 615: recipe tokens
- 620: human interaction tokens
- 625: user behaviour-defined embedding
- 650: first user
- 655: second user
- 660: chart showing group differences

## Claims

1. Method (200) for generating a culinary knowledge base, the method comprising steps of:
- determining user (140) actions during preparation of a dish;
- determining a first cooking recipe, the first cooking recipe comprising preparation steps which are similar to the user (140) actions;
- generating a second cooking recipe, the second cooking recipe comprising preparation steps that match the user's (140) actions for preparing said dish; and
- storing the second cooking recipe as a variant of the first cooking recipe.

2. Method (200) according to claim 1, wherein it is determined that a dissimilarity between the first and second cooking recipe is lower than a first predetermined threshold.

3. Method (200) according to claim 1 or 2, wherein it is determined that a dissimilarity between the first and second cooking recipe is greater than a second predetermined threshold.

4. Method (200) according to claim 2 or 3, wherein each recipe is associated a point in a latent space of an artificial neural network (129); and a dissimilarity between two recipes is determined on the basis of a distance between their associated points in said latent space.

5. Method (200) according to one of the above claims, wherein the first cooking recipe is selected from a repository comprising a plurality of cooking recipes.

6. Method (200) according to one of the above claims, wherein the first recipe is generated in text form using a Large Language Model.

7. Method (200) according to one of the above claims, wherein the user (140) action concerns an ingredient or intermediate product to the dish, an amount or state of the ingredient or intermediate product.

8. Method (200) according to one of the above claims, wherein the user (140) action comprises a processing of an ingredient or intermediate product.

9. Method (200) according to one of the above claims, wherein the user (140) action comprises the use of one or more kitchen appliances (135).

10. Method (200) according to one of the above claims, wherein the second recipe is only stored if the user (140) expresses his contentment with the prepared dish.

11. Method (200) according to one of the above claims, wherein the second recipe is stored with a reference to characteristics of said user (140).

12. Method (200) according to claim 11, especially in combination with one of claims 2 and 3, wherein a dissimilarity between cooking recipes comprises a dissimilarity of associated user (140) characteristics.

13. Kitchen appliance (135), wherein the kitchen appliance (135) comprises:
- a sensor for determining a user (140) action during preparation of a dish;
- a processing means that are adapted to determine, based on determined user (140) actions, a first cooking recipe, the first cooking recipe comprising preparation steps which are similar to the user (140) actions; to generate a second cooking recipe, the second cooking recipe comprising preparation steps that match the user's (140) actions for preparing said dish; and to store the second cooking recipe as a variant of the first cooking recipe.

14. Service, wherein the service is adapted to determine dissimilarity between two cooking recipes on the basis of an artificial neural network.

15. System (100) for generating a culinary knowledge base, the system comprising at least one kitchen appliance (135) according to claim 13, a service according to claim 14 and a storage device for a plurality of cooking recipes.

## Amended claims

### Amended claims in accordance with Rule 137(2) EPC.

1. Method (200) for generating a culinary knowledge base, the method comprising steps of:
- determining user (140) actions during preparation of a dish using electronic processing means;
- determining a first cooking recipe using electronic processing means, the first cooking recipe comprising preparation steps which are similar to the user (140) actions;
- generating a second cooking recipe using electronic processing means, the second cooking recipe comprising preparation steps that match the user's (140) actions for preparing said dish; and
- storing the second cooking recipe as a variant of the first cooking recipe; **characterized in that**
- the user (140) action comprises a processing of an ingredient or intermediate product, wherein processing information is determined on a basis of an observation of the user's actions using a camera or on a basis of operation information of one or more kitchen appliances, which is used by the user and comprises a sensor for determining a user action during preparation of the dish.

2. Method (200) according to claim 1, wherein it is determined that a dissimilarity between the first and second cooking recipe is lower than a first predetermined threshold.

3. Method (200) according to claim 1 or 2, wherein it is determined that a dissimilarity between the first and second cooking recipe is greater than a second predetermined threshold.

4. Method (200) according to claim 2 or 3, wherein each recipe is associated a point in a latent space of an artificial neural network (129); and a dissimilarity between two recipes is determined on the basis of a distance between their associated points in said latent space.

5. Method (200) according to one of the above claims, wherein the first cooking recipe is selected from a repository comprising a plurality of cooking recipes.

6. Method (200) according to one of the above claims, wherein the first recipe is generated in text form using a Large Language Model.

7. Method (200) according to one of the above claims, wherein the user (140) action concerns an amount or state of the ingredient or intermediate product.

8. Method (200) according to one of the above claims, wherein the second recipe is only stored if the user (140) expresses his contentment with the prepared dish.

9. Method (200) according to one of the above claims, wherein the second recipe is stored with a reference to characteristics of said user (140).

10. Method (200) according to claim 9, especially in combination with one of claims 2 and 3, wherein a dissimilarity between cooking recipes comprises a dissimilarity of associated user (140) characteristics.

11. Kitchen appliance (135), wherein the kitchen appliance (135) comprises:
- a sensor for determining a user (140) action during preparation of a dish;
- a processing means that are adapted to determine, based on determined user (140) actions, a first cooking recipe, the first cooking recipe comprising preparation steps which are similar to the user (140) actions; to generate a second cooking recipe, the second cooking recipe comprising preparation steps that match the user's (140) actions for preparing said dish; and to store the second cooking recipe as a variant of the first cooking recipe;
**characterized in that**
- the user (140) action comprises a processing of an ingredient or intermediate product, wherein processing information is determined on a basis of an observation of the user's actions using a camera or on a basis of operation information of the kitchen appliance using the sensor.

12. System (100) for generating a culinary knowledge base, the system comprising at least one kitchen appliance (135) according to claim 11, a service being adapted to determine dissimilarity between two cooking recipes on a basis of an artificial neural network and a storage device for a plurality of cooking recipes.
